# EUROPÄISCHE PATENTSCHRIFT

(11) **EP 0 730 477 B1**
(45) Veröffentlichungstag und Bekanntmachung des Hinweises auf die Patenterteilung: **03.06.1998**
(21) Anmeldenummer: 94931573.3
(22) Anmeldetag: 08.11.1994
(51) Int. Cl.: A61M 16/04

(54) **TRACHEALKANÜLE FÜR DIE MASCHINELLE BEATMUNG TRACHEOTOMIERTER PATIENTEN**
TRACHEAL TUBE FOR ARTIFICIAL RESPIRATION OF TRACHEOSTOMIZED PATIENTS
CANULE TRACHEALE PERMETTANT LA RESPIRATION ARTIFICIELLE DE PATIENTS TRACHEOTOMISES

(30) Priorität: 22.11.1993 DE 4339706
(43) Veröffentlichungstag der Anmeldung: 11.09.1996
(73) Patentinhaber: WILLY RÜSCH AG, D-71394 Kernen (DE)
(72) Erfinder: KEIM, Michael, D-97256 Geroldshausen (DE)
(74) Vertreter: Götz, Georg, Dipl.-Ing.
(86) Internationale Anmeldenummer: EP9403647
(87) Internationale Veröffentlichungsnummer: WO9514499

(56) Entgegenhaltungen:
- US-A- 4 280 492
- US-A- 4 459 984
- US-A- 4 596 248
- US-A- 4 852 565

## Beschreibung

Die Erfindung richtet sich auf eine Trachealkanüle für die maschinelle Beatmung tracheotomierter Patienten mit einer den proximalen, konzentrisch in die Trachea einsetzbaren Teil umgebenden, diese abdichtenden Cuff und einem oberhalb desselben angeordneten, gekrümmten Bereich sowie mit einer in die Kanüle von deren distalen Ende her konzentrisch einsetzbaren Innenkanüle, wobei in der Kanüle oberhalb des die Trachea abdichtenden Cuffs, sowie etwa in der Verlängerung der Längsachse des umgebogenen, in die Trachea einsetzbaren Teils eine Exspirationsöffnung vorgesehen ist.

Zur Vermeidung der Gefahr des Erstickens muß anstelle einer Intubation in bestimmten Fällen eine Tracheotomie durchgeführt werden, um einen Patienten maschinell beatmen zu können. Hierbei wird nach dem Öffnen der Trachea eine eigens dafür vorgesehene, etwa 10 cm lange Kanüle mit einem Innendurchmesser von etwa 1 cm eingesetzt. In diese Kanüle kann sodann eine sogenannte Seele, d.h., eine zweite, innere Kanüle vom distalen Ende her eingeführt werden. Diese Seele ist an ihrem äußeren Ende mit einem Verbindungsstück, einem sogenannten Konnektor, zum Anschluß an ein Beatmungsgerät versehen.

Im Gegensatz zur Eigenatmung, die durch die Aktivität des Zwerchfells und der Intercostalmuskulatur einen Ausgleich zwischen atmosphärischem Druck und intrapulmonalem Druck herstellt, wird bei der maschinellen Beatmung ein bestimmtes Luftvolumen durch die Trachealkanüle und die Trachea in die Lunge hineingepresst. Dies führt zu einem gegenüber dem atmosphärischen Druck erhöhten Druck innerhalb der Lunge.

Damit der hierfür notwendige Druck aufgebaut werden kann, muß die Trachea mit einem Ballon (Cuff), der unterhalb des Larynx in der Trachea liegt, abgedichtet werden. Diese Abdichtung sorgt dafür, daß die in der Lunge enthaltene Luft ausschließlich durch die Trachealkanüle entweichen kann, sobald im Beatmungsgerät ein entsprechendes Ventil geöffnet ist.

Somit werden bei einer maschinellen Beatmung durch eine Trachealkanüle die oberhalb des die Trachea abdichtenden Cuffs befindlichen Luftwege vollständig umgangen. Das Exspirationsvolumen kann somit nicht mehr durch den Larynx strömen. Zur Erzeugung von Tönen muß aber das Exspirationsvolumen durch die geschlossene Stimmritze gepresst werden, damit die Stimmbänder zu Schwingungen angeregt werden können. Fehlt die Möglichkeit, mit Hilfe der Stimmbänder die Luftsäule in Schwingungen zu versetzen, kann der betreffende Patient keine Vokale aussprechen. Denn diese entsprechen vom Larynx erzeugten Tönen unterschiedlicher Höhe, Stärke und Klangfarbe. Zwar kann der Patient in einem solchen Fall noch mit Hilfe von Lippen, Zähnen, Zunge und Gaumen Geräusche erzeugen, somit einzelne Konsonanten hervorrufen, aber ohne Vokale ist eine Verständigung völlig ausgeschlossen.

Die US-PS 4,852,565 beschreibt eine Trachealkanüle zur maschinellen Beatmung. Diese weist eine in die Trachea einsetzbare Außenkanüle sowie eine in die Außenkanüle einführbare Seele mit einer Innenkanüle und einem Konnektor zum Anschluß an ein Beatmungsgerät auf. Zur Übung der Eigenatmung ist in der Außenkanüle oberhalb eines die Trachea abdichtenden Cuffs sowie etwa in der Verlängerung der Längsachse des umgebogenen, in die Trachea einsetzbaren Teils eine oder mehrere Öffnungen vorgesehen. Um eine Atmung durch den oberen Teil der Trachea und insbesondere durch den Larynx zu ermöglichen, muß jedoch die Innenkanüle, welche die betreffenden Öffnungen in der Außenkanüle in eingeschobenem Zustand abdichtet, aus der Außenkanüle herausgezogen werden. Sodann wird das distale Ende der Außenkanüle vermittels eines Propfens verschlossen, so daß der Patient zur Eigenatmung gezwungen ist und sowohl das Inspirations- als auch das Exspirationsvolumen durch den Larynx strömen muß, so daß der Patient in der Lage ist, wie gewohnt zu sprechen. Da hierfür jedoch eine vollständige Abtrennung vom Beatmungsgerät notwendig ist, ist die Anwendung auf Patienten beschränkt, deren Genesungsprozeß schon weit vorangeschritten ist, so daß sie zumindest zeitweise zur Eigenatmung fähig sind. In dem oftmals weitaus längeren Zeitraum, während dessen eine maschinelle Beatmung für den Patienten unabdingbar ist, ist mit der vorbekannten Anordnung jedoch das Erzeugen von Tönen nicht möglich.

Weiterhin beschreibt die DE-PS 37 20 482 eine Trachealkanüle mit einem gekrümmten Rohrstück, das durch eine Halsöffnung in die Trachea eines Patienten eingeführt wird. Innerhalb des gebogenen Rohrstücks befindet sich ein durch eine Membran umschlossenes Volumen, welches über einen dünnen Schlauch mit einem externen Balg verbunden ist und durch Betätigen desselben ballonartig aufgebläht werden kann, so daß die exotracheale Mündung der Kanüle vollständig verschlossen werden kann. Die betreffende Trachealkanüle ist jedoch wie die aus der US-PS 4,852,565 beschriebene Kanüle nur für selbstatmende Patienten geeignet, da nach Verschließen der exotrachealen Kanülenmündung vermittels des ballonartigen Ventils eine maschinelle Beatmung vollständig blockiert würde. Im Gegensatz zu den gattungsgemäßen Trachealkanülen für die maschinelle Beatmung weist die aus der DE-PS 37 20 482 vorbekannte Kanüle keinen Cuff auf, damit der Patient bei blockierter Kanülenmündung über den Larynx atmen kann. Demzufolge fehlt bei dieser Kanüle auch eine Exspirationsöffnung völlig, da sich das entsprechende Luftvolumen bei verschlossener Kanülenöffnung nicht durch, sondern um die Kanüle bewegt. Aufgrund der vielfälltigen konstruktiven Unterschiede eignet sich diese vorbekannte Anordnung nicht für maschinell beatmete Patienten und stellt somit einen weiter ab liegenden Stand der Technik da.

Aus diesem Nachteil vorbekannter Anordnungen resultiert das der Erfindung zugrundeliegende Problem, eine Trachealkanüle so auszugestalten, daß trotz der maschinellen Beatmung mit dem Larynx Töne erzeugt werden können, so daß der Patient auch Vokale sprechen und sich somit verständigen kann.

Zu diesem Zweck sieht die Erfindung bei einer Trachealkanüle für die maschinelle Beatmung tracheotomierter Patienten mit einer den proximalen, konzentrisch in die Trachea einsetzbaren Teil umgebenden, diese abdichtenden Cuff und einem oberhalb desselben angeordneten, gekrümmten Bereich sowie mit einer in die Kanüle von deren distalen Ende her konzentrisch einsetzbaren Innenkanüle, wobei daß in der Kanüle oberhalb des die Trachea abdichtenden Cuff sowie etwa in der Verlängerung der Längsachse des umgebogenen, in die Trachea einsetzbaren Teils eine Exspirationsöffnung vorgesehen ist, vor, daß die Innenkanüle in ihrem unterhalb der Exspirationsöffnung gelegenen Bereich eine durch eine als Ventil wirkende, elastische Membran zumindest teilweise abgedeckte Ausnehmung aufweist sowie einen querschnittlich verjüngten Bereich an ihrem proximalin Ende. Während der Inspirationsphase wird ein bestimmtes Luftvolumen von dem Beatmungsgerät durch die vom distalen Ende her in die Trachealkanüle eingesetzte Innenkanüle, den proximalen Bereich der Trachealkanüle und die Trachea in die Pulmones gepreßt. Die eingepreßte Luft weist einen Überdruck im Verhältnis zum atmosphärischen Umgebungsdruck auf. Diese Druckdifferenz besteht insbesondere zwischen dem Innenraum der Seele und dem oberhalb des abdichtenden Cuff befindlichen Bereichs der Trachea. Da eben in diesem Bereich unterschiedlichen Drucks in der Trachealkanüle eine Exspirationsöffnung angeordnet ist, wirkt diese Druckdifferenz direkt auf die darunter befindliche Innenkanüle ein. Diese ist in dem betreffenden Bereich als elastische Membran ausgebildet, welche sich je nach Richtung der Druckdifferenz nach außen dehnt oder nach innen zusammenzieht. Wird von dem Beatmungsgerät unter Überdruck stehende Luft in die Pulmones gepreßt, dehnt sich die an der Innenkanüle befindliche Membran im Bereich der Exspirationsöffnung der Trachealkanüle nach außen und legt sich dabei entlang des Umfangsrandes der Exspirationsöffnung an die innere Oberfläche der Kanüle an. Hierdurch wird die Exspirationsöffnung abgedichtet und verhindert, daß das eingepresste Luftvolumen, anstelle in die Pulmones zu strömen, direkt über die oberen Luftwege entweichen kann.

Sobald im Beatmungsgerät dagegen das Ausströmventil geöffnet wird, setzt die Exspirationsphase ein. Das in den Pulmones befindliche, unter Überdruck stehende Luftvolumen strömt hierbei zunächst durch den unteren Bereich der Trachea, die Trachealkanüle sowie den Innenraum der Innenkanüle und den Konnektor zum Beatmungsgerät, von wo es durch das Ausströmventil in die Umgebung entweicht. Durch den querschnittlich verjüngten Bereich am proximalen Ende der Innenkanüle wird die Strömungsgeschwindigkeit der ausströmenden Luft begrenzt, so daß das Exspirationsvolumen mit verringerter Geschwindigkeit ausströmt. Dadurch bleibt der Überdruck in den Pulmones aufrechterhalten, während oberhalb des querschnittlich verjüngten Bereichs innerhalb der Innenkanüle die Luft nahezu unbehindert zum Beatmungsgerät strömen kann. Daher wird in diesem Bereich der Luftdruck etwa auf den atmosphärischen Druck reduziert, so daß sich die elastische Membran nach Wegfall der dehnenden Druckdifferenz in ihre ursprüngliche Lage zusammenzieht. In diesem Zustand liegt sie aber im Bereich der Umfangsränder der Exspirationsöffnung nicht mehr an der Innenseite der Trachealkanüle an. Hierdurch wird ein zweiter Strömungspfad für einen Teil des Exspirationsvolumens frei, nämlich durch die Trachea, den proximalen Bereich der Trachealkanüle, den Zwischenraum zwischen dem verjüngten Bereich der Innenkanüle und der Innenwand der Trachealkanüle, deren Exspirationsöffnung und den oberen Bereich der Lufwege, insbesondere den Larynx, nach außen. Der durch den Larynx strömende Teil des Exspirationsvolumens ist dabei in der Lage, über die Stimmbänder Schwingungen der Luftsäule herbeizuführen, so daß der betreffende Patient wie gewohnt Töne erzeugen und diese zu Vokalen umformen kann. Während der Exspirationsphase ist es dem Patienten demnach möglich, sich wie gewohnt durch Sprache mit seiner Umgebung zu verständigen. Darüberhinaus werden auch die oberen Luftwege erwärmt, befeuchtet und gereinigt. Schließlich versetzt der über die oberen Luftwege strömende Anteil des Exspirationsvolumens den betreffenden Patienten in die Lage, Gerüche wahrzunehmen, da das Exspirationsvolumen über die Riechplatte strömen kann.

Eine vorteilhafte Weiterbildung der Erfindung besteht darin, daß die durch eine elastische Membran abgedeckte Ausnehmung der Innenkanüle sich schlitzartig bis zu deren proximaler Stirnseite fortsetzt. Die Ausnehmung bildet demnach einen in die proximale Stirnseite der Innenkanüle mündenden Schlitz. Dies hat den Vorteil, daß sich je nach Strömungsrichtung der innerhalb der Innenkanüle befindlichen Luft der Querschnitt des proximalen Endes der Innenkanüle verändern kann. Insbesondere ist es dadurch möglich, daß sich die Membran in diesem Bereich der Innenkanüle während der Inspirationsphase unter dem Strömungsdruck der in die Pulmones strömenden Luft nach außen dehnt, so daß die Luftströmung weniger behindert wird.

Andererseits zieht sich die Membran in ihrem proximalen Bereich bei Fehlen einer Strömung zusammen und nimmt dabei eine Lage ein, bei der sie die Schlitzränder ohne merkliche Wölbung direkt miteinander verbindet. Die Membran bildet hierbei querschnittlich etwa eine Sekante, welche den kreisförmigen Querschnitt der Innenkanüle zwischen den beiden Schlitzrändern in einen innerhalb der Innenkanüle liegenden Teil sowie einen zwischen der Membran und der Innenwand der Kanüle verbleibenden Zwischenraum unterteilt. Hierdurch ergibt sich zu Beginn der Exspirationsphase eine Verengung des Strömungsquerschnitts, an welchem nahezu der gesamte, die Exspirationsströmung hervorrufende Überdruck in den Pulmones abfällt.

Durch die hieraus resultierende Druckverminderung im Bereich der Kanülenkrümmung zieht sich die Membran auch in ihrem oberen, direkt unterhalb der Exspirationsöffnung gelegenen Bereich auf den ihr eigenen Querschnitt zusammen und gibt dabei die Exspirationsöffnung frei. Das Ablösen der Membran von der Exspirationsöffnung wird dabei zusätzlich dadurch gefördert, daß innerhalb des in der Zwischenwand der Kanüle und der Membran verbleibenden Zwischenraums zumindest anfänglich derselbe Überdruck herrscht wie in den Pulmones, während unterhalb der Membran, also innerhalb der Seele nach Einsetzen der Exspirationsströmung kaum noch ein erhöhter Druck herrscht. In dieser Phase wirkt also der Überdruck an der Membran in genau entgegengesetzter Richtung und drückt daher diese noch weiter von der Exspirationsöffnung weg. Die Exspirationsöffnung wird dadurch vollständig freigegeben und das Exspirationsvolumen nimmt zum Großteil den Weg über die oberen Luftwege.

Es hat sich als günstig erwiesen, daß die durch eine elastische Membran abgedeckte schlitzartige Ausnehmung der Innenkanüle am proximalen Ende querschnittlich etwa den halben Wölbungsumfang des Seelenquerschnitts einnimmt. Bei einer derartigen Ausgestaltung ist die Wirkung der elastischen Membran maximal. Sie kann sich einerseits während der Inspirationsphase bereits bei einer geringen Strömungsgeschwindigkeit ausdehnen und somit nahezu den gesamten Seelenquerschnitt freigeben; andererseits verengt sie in entspanntem Zustand den Strömungsquerschnitt zu Beginn der Exspirationsphase etwa auf den halben Seelenquerschnitt, was für eine Druckreduzierung im oberen Membranbereich völlig ausreichend ist.

Nach Einsetzen der Exspirationsströmung über die oberen Luftwege dehnt sich die Membran in ihrem unteren Bereich je nach den Druckverhältnissen innerhalb des distalen Bereich der Innenkanüle und der oberen Luftwege der Patienten, wobei jeweils der Strömungspfad mit dem höheren Druck erweitert wird. Dies hat den Vorteil, daß beim Sprechen des Patienten, wenn das Exspirationsvolumen durch die Stimmritze gepreßt werden muß und dabei einen höheren Luftwiderstand erfährt, durch den hervorgerufenen Überdruck die Membran nach innen gepreßt wird, so daß ein relativ großer Teil der Luftströmung seinen Weg über den Larynx nimmt und dabei die Schallerzeugung unterstützt. Die Membran hat somit die Wirkung, daß gerade in dem Augenblick, wenn der Patient sprechen möchte, besonders viel Luft durch den Larynx geleitet wird.

Weiterhin ist es vorteilhaft, daß die elastische Membran in ihrem proximal zur Exspirationsöffnung gelegenen Bereich in weitgehend entspanntem Zustand einen gegenüber dem Wölbungsumfang des Querschnitts der Innenkanüle verringerten Umfang aufweist. Hierdurch ist ausgeschlossen, daß die Membran an der Innenseite der Trachealkanüle einerseits als auch an der Innenseite der Innenkanüle andererseits anliegt und einen der beiden Strömungspfade vollständig verschließt. Somit ist sichergestellt, daß auch bei vollständig verschlossenen oberen Luftwegen eine Exspiration über das Beatmungsgerät stattfinden kann. Eine Komplikation, die sich daraus ergeben könnte, daß die Membran zufällig zu Beginn der Exspirationsphase an der Innenseite der Innenkanüle anliegt und somit bei verschlossenen oberen Luftwegen keine Ausatmung möglich ist, was im Hinblick auf eine Erstickungsgefahr zu beachten ist, wird somit völlig ausgeschlossen. Die Membran bewegt sich mit ihrem proximalen Ende bei der erfindungsgemäßen Dimensionierung nie vollständig bis zur Innenseite der Kanüle oder der Innenkanüle. Während der Exspirationsphase sind somit immer beide Strömungswege geöffnet, was die Sicherheit des Patienten sogar gegenüber herkömmlichen Trachealkanülen erhöht, da bei einem Versagen des Ausströmventils im Beatmungsgerät zumindest noch der Strömungspfad über die oberen Luftwege zur Verfügung steht.

Weiterhin sieht die Erfindung vor, daß die elastische Membran in ihrem unterhalb der Exspirationsöffnung gelegenen Bereich in entspanntem Zustand etwa einen dem Wölbungsumfang des Querschnitt der Innenkanüle entsprechenden Umfang aufweist. Eine solche Dimensionierung hat zur Folge, daß die Exspirationsöffnung bereits zu Beginn der Inspirationsphase vollständig geschlossen wird, so daß vermittels des Beatmungsgeräts der Überdruck in Kanüle, Trachea und Pulmones langsam und gleichmäßig aufgebaut werden kann, was dem natürlichen Atmungsvorgang entspricht. Andererseits kann die Membran in diesem Bereich beim Freigeben der Exspirationsöffnung entsprechend den Druckverhältnissen unproblematisch von der konvexen, an der Innenseite der Kanüle anliegenden Wölbung in eine konkave, weit in den Innenraum der Seele hineinreichende Wölbung umgestülpt werden, so daß - insbesondere während des Sprechens - nahezu die gesamte Luftströmung ohne nennenswerten Druckabfall bis zur Stimmritze gelangen kann und somit auch laute Töne möglich sind. Denn die Tonstärke hängt von der Amplitude der erzeugten Schwingungen ab, und diese wiederum von dem Druck, mit dem die Ausatmungsluft durch die Stimmritze gepreßt wird.

Es liegt im Rahmen der Erfindung, daß die Innenkanüle ein radiales Anschlagelement zur Begrenzung der Einschubbewegung in die Kanüle aufweist, das insbesondere bei Trachealkanülen mit einem Konnektor, der mit der Innenkanüle verbunden ist und als Anschlußstück für ein Beatmungsgerät dient, vorzugweise an dem Konnektor angeformt ist. Durch die Begrenzung der Einschubbewegung der Innenkanüle kann eine definierte Lage derselben problemlos eingestellt werden, so daß eine Fluchtung zwischen Exspirationsöffnung und schlitzartiger Ausnehmung sichergestellt ist.

Schließlich entspricht es der Lehre der Erfindung, daß auf dem distalen, oberhalb der Krümmung gelegenen Bereich der Kanüle ein ringförmiger Haltesteg verschieb- sowie fixierbar angeordnet ist. Die erfindungsgemäße Trachealkanüle wird nach dem Einsetzen in die Trachea vermittels eines den Hals des Patienten umschlingenden Kanülenbands fixiert. Dieses wird in diametral gegenüberliegenden Öffnungen an einer Halteplatte eingehängt, welche den distalen Bereich der Kanüle umschließt. Durch die Elastizität des den Hals des Patienten umschließenden Haltebandes wird der Haltesteg fest an den Hals des Patienten angepreßt. Bei herkömmlichen Trachealkanülen, wo der Haltesteg einstückig mit der Kanüle ausgebildet ist, wirkt sich dieser Umstand nicht negativ aus, da die Kanüle selbst elastisch ist und entsprechend dem Halsumfang des Patienten unterschiedlich weit in die Trachea eindringen kann. Bei der erfindungsgemäßen Trachealkanüle muß die Lage der Halteplatte dagegen an den Halsumfang des Patienten angepaßt werden, damit die in dem gekrümmten Bereich angeordnete Exspirationsöffnung genau unterhalb des oberen Bereichs der Trachea liegt. Nur in diesem Fall ist ein Strömungspfad über die oberen Luftwege möglich, der eine Artikulation erlaubt. Zur Realisierung eines verschieb- sowie fixierbaren Haltestegs ist es beispielsweise möglich, den Innendurchmesser der das distale Kanülenende aufnehmenden Öffnung des Haltestegs geringfügig kleiner zu wählen als den Außenumfang der Kanüle, so daß der Haltesteg durch elastische Aufweitung auf die Kanüle aufgeschoben ist. Die daraus resultierende, elastische Anpreßkraft führt zu einer hohen Reibungskraft zwischen Kanüle und Haltesteg, so daß dieser unter normalen Bedingungen unverrückbar auf der Kanüle festsitzt. Demnach kann - beispielsweise während einer Operation - der Haltesteg an den Halsumfang des Patienten angepaßt werden. Es ist beispielsweise auch denkbar, auf der Kanüle Markierungen anzubringen, auf die der Haltesteg je nach dem Halsumfang des Patienten zu plazieren ist.

Weitere Einzelheiten, Merkmale und Vorteile auf der Basis der Erfindung ergeben sich aus der nachfolgenden Beschreibung einer bevorzugten Ausführungsform der Erfindung sowie anhand der Zeichnung. Hierbei zeigen:
- Fig. 1: eine Seitenansicht einer erfindungsgemäßen Trachealkanüle, teilweise aufgeschnitten,
- Fig. 2: eine perspektivische Ansicht einer erfindungsgemäßen, in die Trachealkanüle gemäß Fig. 1 einzusetzenden Seele,
- Fig. 3: einen Längsschnitt durch die Seele gemäß Fig. 2,
- Fig. 4: die Trachealkanüle gemäß Fig. 1 mit eingesetzter Seele gemäß Fig. 3 im Längsschnitt während der Einatmungsphase sowie
- Fig. 5: dieselbe Anordnung wie in Fig. 4 während der Ausatmungsphase.

Der Aufbau der erfindungsgemäßen Trachealkanüle 1 ist prinzipiell ähnlich dem aus dem Stand der Technik bekannter Trachealkanülen. Die Kanüle 1 besteht aus elastischem, physiologisch verträglichen Werkstoff. Sie weist die Form einer gebogenen Röhre 2 kreisförmigen Querschnitts auf. Diese Röhre 2 hat einen Innendurchmesser von etwa 1 cm und eine Gesamtlänge von etwa 10 cm. Die etwa rechtwinklige Krümmung 3 der Kanülenröhre 2 befindet sich in deren mittleren Bereich und unterteilt die Kanülenröhre 2 in einen unteren, in die Trachea eines Patienten einsetzbaren Bereich 4 und einen oberen, durch den Luftröhrenschnitt hindurchtretenden Bereich 5.

Zur Fixierung des distalen Endes 6 der Trachealkanüle 1 am Hals des Patienten dient ein ringförmiger Haltesteg 7, welcher in seinen seitlichen Bereichen zwei einander diametral gegenüberliegende Laschen mit je einer Ausnehmung zum Einhängen eines Befestigungsbands aufweist.

Damit die durch die maschinelle Beatmung eingepreßte Luft nicht durch einen möglicherweise zwischen der Außenseite des in die Trachea eingesetzten Bereichs 4 der Kanüle 1 und der inneren Oberfläche der Trachea verbleibenden Zwischenraum in den oberen Bereich der Trachea gelangen und von dort über die oberen Luftwege entweichen kann, ist im unteren Bereich 4 des Kanülentubus 2 ein diesen umgebender, mit elastischem Schaumstoff 8 gefüllter Ballon 9 angeordnet. Diese als Cuff 10 bezeichnete Anordnung dichtet einen Zwischenraum zwischen Kanülentubus 4 und Trachea luftdicht ab.

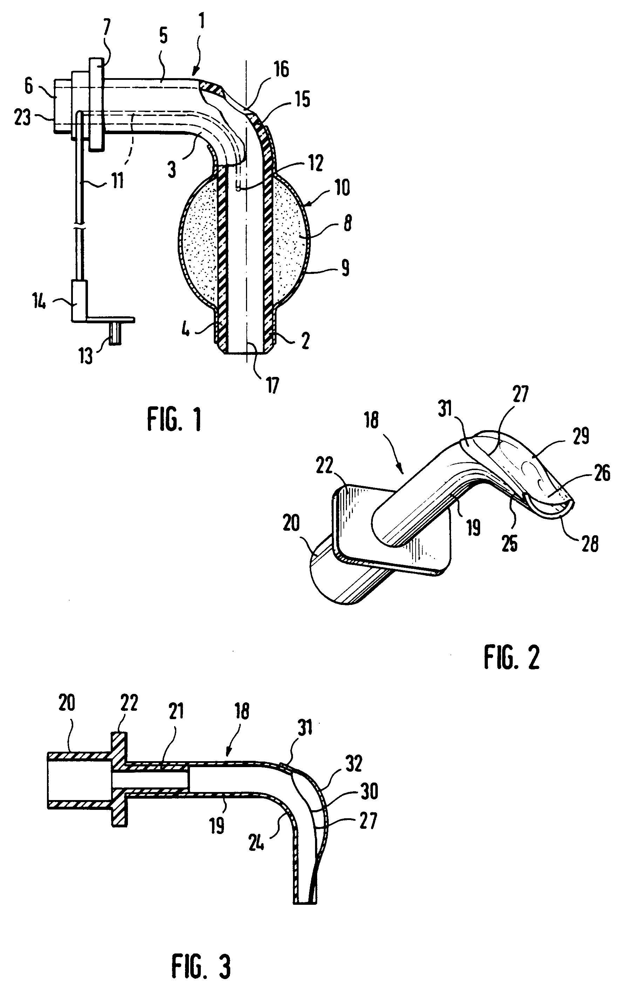

Um den Cuff 10 während des Einsetzens durch den Luftröhrenschnitt schieben zu können, kann in dem mit elastischem Schaumstoff 8 gefüllten, tubusförmigen Bereich zwischen Kanülentubus 4 und Ballonhülle 9 ein Vakuum erzeugt werden, so daß der atmosphärische Luftdruck entgegen der elastischen Kraft des Schaumstoffs 8 die Ballonhülle 9 gegen den Kanülentubus 4 preßt. Zur Erzeugung eines solchen Vakuums dient ein innerhalb des oberen Bereichs 5 der Kanüle 1 verlaufender Schlauch 11, dessen proximales Ende 12 innerhalb des Cuffs 10 durch eine den Kanülentubus 4 durchsetzende, in Fig. 1 außerhalb der Zeichenebene liegende Ausnehmung in den mit Schaumstoff 8 gefüllten Hohlraum zwischen Kanülentubus 4 und Ballonhülle 9 mündet. Der Schlauch 11 tritt am distalen Ende 6 der Kanüle 1 nach außen und endet an einem durch einen Propfen 13 verschließbaren Mundstück 14. Ein vor dem Einsetzen der Trachealkanüle 1 innerhalb des Cuffs 10 erzeugtes Vakuum kann durch Verschließen des Mundstücks 14 mittels des Pfropfens 13 beliebig lang aufrechterhalten werden. Wird nach dem Einsetzen der Kanüle 1 der Pfropfen 13 aus dem Mundstück 14 gezogen, vermag Luft durch den Schlauch 11 in den Cuff 10 einzudringen, so daß dieser sich unter der Wirkung des eingeschlossenen, elastischen Schaumstoffs 8 ausdehnt und dabei die Trachea luftdicht abschließt.

Um dem Patienten dennoch eine Artikulation zu ermöglichen, ist an dem bezüglich der Krümmung 3 radial außen gelegenen Bereich 15 des Tubus 2 eine etwa kreisförmige oder ovale Exspirationsöffnung 16 vorgesehen. Aufgrund der Krümmung 3 der Kanüle 1 befindet sich die Exspirationsöffnung 16 etwa im Bereich der Längsachse 17 des proximalen, in die Trachea eingesetzten Bereichs 4 der Kanüle 1. Die Exspirationsöffnung 16 liegt daher genau innerhalb des oberen Bereichs der Trachea, der mit demjenigen Teil, in den der Kanülentubus 4 eingesetzt ist, fluchtet. Die Exspirationsöffnung 16 stellt damit eine Verbindung zu den oberen Luftwegen dar.

Damit beim Einsetzen der Trachealkanüle 1 die Exspirationsöffnung 16 genau unterhalb des oberen Trachealbereichs plaziert werden kann, ist der Haltesteg 7 entlang des distalen Bereichs der Trachealkanüle 1 linear verschiebbar angeordnet. Durch seine Elastizität ist er in der Lage, an jeder gewünschten Stelle eine hohe Reibungskraft zu erzeugen und somit nahezu unverrückbar festzuklemmen. Hierdurch kann unterschiedlichen Halsweiten tracheotomierter Patienten Rechnung getragen werden.

Um die Exspirationsöffnung 16 je nach Atmungsphase differenziert öffnen oder schließen zu können, ist eine speziell geformte Seele 18 vorhanden. Die Seele 18 besteht aus einem in den distalen Bereich 6, 5 der Kanüle 1 als zweite, innenliegende Kanüle einschiebbaren Bereich 19 aus ebenfalls elastischem Kunststoff, sowie aus einem Konnektor 20, der als Anschlußstück für ein Beatmungsgerät dient. Die geringfügig elastische Innenkanüle 19 ist unter radialer Dehnung auf einen entsprechenden Fortsatz 21 des Konnektors 20 aufgeschoben, so daß aufgrund der hohen Reibungskraft eine feste Verbindung zwischen Innenkanüle 19 und Konnektor 20 gegeben ist.

Die Innenkanüle 19 wird so weit in den distalen Bereich 6, 5 der Trachealkanüle 1 eingeschoben, bis ein radiales Anschlagelement 22 des Konnektors 20 an der distalen Stirnseite 23 der Trachealkanüle 1 anliegt. Hierbei paßt sich die Krümmung 24 der Innenkanüle 19 an die Krümmung 3 der Trachealkanüle 1 an.

Wie man am deutlichsten aus der Fig. 2 ersehen kann, weist die Innenkanüle 19 an ihrem proximalen Ende 25 einen Längsschlitz 26 auf, welcher sich an der bezüglich der Krümmung 24 radial außen liegenden Seite 27 von der proximalen Stirnseite 28 bis nahezu über den gesamten Bereich der Krümmung 24 erstreckt. Der Umfang der Ausnehmung 26 entspricht etwa einem U. Die maximale Schlitzbreite der Ausnehmung 26 ist etwa gleich dem Durchmesser der Innenkanüle 19.

Diese schlitzartige Ausnehmung 26 in der Mantelfläche der zwar geringfügig elastischen, insgesamt aber doch relativ steifen Innenkanüle 19 wird durch eine hauchdünne, hochelastische Membran 29 abgedeckt, welche entlang eines den Umfangsrand 30 der Ausnehmung 26 überlappenden Bereichs 31 mit der Innenkanüle 19 luftdicht verklebt ist. Die Membran 29 ist im Bereich 32 der Krümmung 24 im spannungs-freien Zustand nach außen gewölbt, befindet sich also etwa dort, wo der durch den Schlitz 26 herausgetrennte Bereich der Innenkanüle 19 läge. Im Bereich des proximalen Endes 25 der Innenkanüle 19 nimmt diese Wölbung zunächst kontinuierlich ab, um schließlich knapp oberhalb der Stirnseite 28 etwa gestreckt zwischen den beiden einander gegenüberliegenden Umfangsrändern 30 der schlitzförmigen Ausnehmung 26 zu verlaufen. In Höhe der Stirnseite 28 nimmt die Wölbung dagegen wieder zu. Aufgrund der inneren Spannungen innerhalb der Membran 29 stülpt diese sich im oberen Bereich 32 nach außen, während sie im Bereich der Stirnseite 28 geringfügig nach innen gewölbt ist.

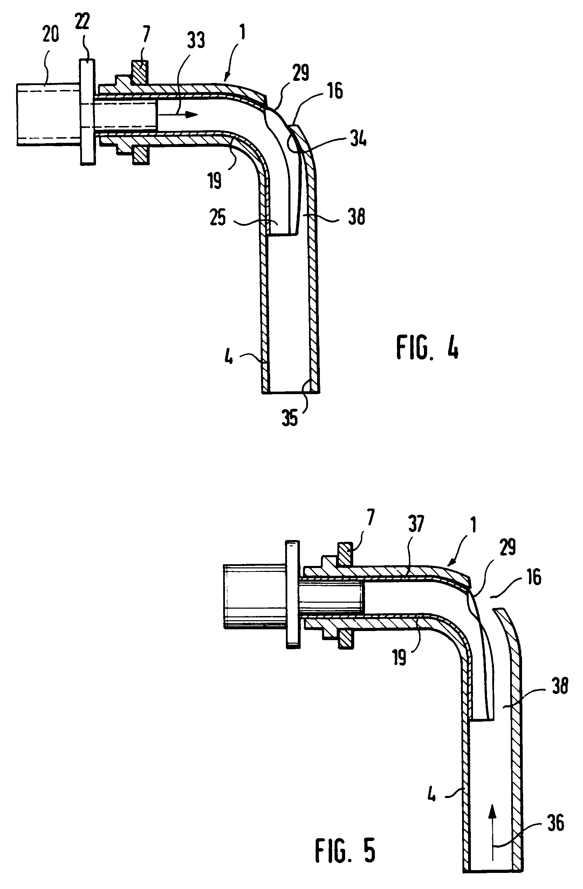

Fig. 4 zeigt die beiden, ineinander geschobenen Kanülen 1, 19 während der Inspirationsphase eines tracheotomierten, maschinell beatmeten Patienten. Aufgrund des von dem nicht dargestellten Beatmungsgerät erzeugten Überdrucks gegenüber den entleerten Pulmones bildet sich eine zum proximalen Ende der Kanülen 1, 19 gerichtete Strömung 33 aus. Die zur Füllung der Pulmones erforderliche Ausdehnung des Brustkorbs des Patienten wird einerseits durch den äußeren Luftdruck, andererseits durch das Eigengewicht des Brustkorbs erschwert. Daher breitet sich der von dem Beatmungsgerät erzeugte Überdruck durch den Konnektor 20, die Innenkanüle 19 und den proximalen Bereich 4 der Trachealkanüle 1 und die Trachea bis in die Pulmones aus.

Wegen des durch die Membran 26 verengten Bereichs 25 der Innenkanüle 19 entsteht in diesem Bereich ein Luftstau, so daß wegen des daraus resultierenden Überdrucks gegenüber dem proximalen Bereich 4 der Trachealkanüle 1 eine bezüglich der Krümmung 24 radial nach außen gerichtete Dehnungskraft auf die Membran 29 einwirkt. Dies führt dazu, daß diese Membran 29 entlang des inneren Umfangsrandes 34 der Exspirationsöffnung 16 an der Innenseite 35 der Trachealkanüle 1 dichtend anliegt. Somit wird die Exspirationsöffnung 16 verschlossen und verhindert, daß das von dem Beatmungsgerät während der Inspirationsphase in die Luftwege hineingepreßte Luftvolumen durch die Exspirationsöffnung 16 entweichen kann.

Die Exspirationsöffnung 16 bleibt so lange verschlossen, solange in den miteinander in Verbindung stehenden Luftwegen gegenüber dem atmosphärischen Druck erhöhter Druck herrscht. Wird dagegen in dem Beatmungsgerät ein Ausströmventil geöffnet, kann sich eine entgegengesetzt gerichtete Strömung 36 ausbilden, wie Fig. 5 zu entnehmen ist. Während innerhalb des distalen Bereichs 37 der Innenkanüle 19, dem Konnektor und dem Beatmungsgerät der Überdruck sehr schnell abgebaut wird, da das enthaltene Luftvolumen, ohne einen Widerstand zu finden, ausströmen kann, sinkt der in den Pulmones, der Trachea und im proximalen Bereich 4 der Trachealkanüle 1 herrschende Überdruck nur relativ langsam ab, da die Strömung 36 in diesem Bereich durch die Verjüngung 25 des Strömungsquerschnitts stark verlangsamt wird. Dies führt dazu, daß der in dem Zwischenraum 38 zwischen der Membran 29 und der Innenseite 35 des Kanülentubus 4 herrschende Druck größer wird als der durch die Abströmung stärker reduzierte Druck innerhalb der Innenkanüle 19. Infolge dieser Druckdifferenz zieht sich die Membran zusammen und wird schließlich sogar in entgegengesetzt, also konkav gewölbter Richtung umgestülpt. Hierbei löst sie sich von den Umfangsrändern 34 der Exspirationsöffnung 16, so daß ein Luftkanal über die oberen Luftwege und insbesondere den Larynx geöffnet wird. Infolgedessen ist es dem Patienten möglich, mit Hilfe des durch die Stimmritze gepreßten Exspirationsvolumens Töne zu erzeugen, diese zu Vokalen zu formen und durch Hinzufügen der betreffenden Konsonanten sich wie gewohnt zu verständigen.

Wenn beim Sprechen die Stimmritze verengt wird, reduziert sich im Larynx der Strömungsquerschnitt, so daß sich der in den Pulmones enthaltene Überdruck nahezu unverändert bis zur Stimmritze ausbreiten kann. Durch diesen Überdruck, der insbesondere auch in dem Bereich 38 zwischen Membran 29 und Innenwand 35 des Kanülentubus 4 herrscht, wird die Membran 29 vollständig umgestülpt und eventuell sogar in entgegengesetzter Richtung gedehnt, so daß der innerhalb der Innenkanüle 19 verbleibende Strömungspfad ebenfalls stark verengt wird und nur eine geringfügige Luftmenge durch das Beatmungsgerät entweicht. Somit steht dem Patienten während des Sprechens nahezu der vollständige Lungendruck zur Verfügung, der darüber hinaus besonders lange aufrechterhalten bleibt, da nur ein geringer Anteil durch das Beatmungsgerät strömt. Somit ist es dem Patienten möglich, auch längere Sätze während einer Exspirationsphase zu sprechen.

## Patentansprüche

1. Trachealkanüle für die maschinelle Beatmung tracheotomierter Patienten mit einer den proximalen, konzentrisch in die Trachea einsetzbaren Teil (4) umgebenden, diese abdichtenden Cuff (10) und einem oberhalb desselben angeordneten, gekrümmten Bereich (3) sowie mit einer in die Kanüle (1) von deren distalen Ende (6) her konzentrisch einsetzbaren Innenkanüle (19), wobei in der Kanüle (1) oberhalb des die Trachea abdichtenden Cuffs (10) sowie etwa in der Verlängerung der Längsachse (17) des umgebogenen, in die Trachea einsetzbaren Teils (4) eine Exspirationsöffnung (16) vorgesehen ist, dadurch gekennzeichnet, daß die Innenkanüle (19) in ihrem unterhalb der Exspirationsöffnung (16) gelegenen Bereich eine durch eine als Ventil wirkende, elastische Membran (29) zumindest teilweise abgedeckte Ausnehmung (26) sowie einen querschnittlich verjüngten Bereich an ihrem proximalen Ende (25) aufweist.

2. Trachealkanüle nach Anspruch 1, dadurch gekennzeichnet, daß die durch eine elastische Membran (29) abgedeckte Ausnehmung (26) der Innenkanüle (19) sich schlitzartig bis zu deren proximaler Stirnseite (28) fortsetzt.

3. Trachealkanüle nach Anspruch 2, dadurch gekennzeichnet, daß die durch eine elastische Membran (29) abgedeckte, schlitzartige Ausnehmung (26) der Innenkanüle (19) am proximalen Ende (25) querschnittlich etwa den halben Wölbungsumfang des Querschnitts der Innenkanüle (19) einnimmt.

4. Trachealkanüle nach Anspruch 3, dadurch gekennzeichnet, daß die elastische Membran (29) in ihrem proximal zur Exspirationsöffnung (16) gelegenen Bereich in weitgehend entspanntem Zustand einen gegenüber dem Wölbungsumfang des Querschnitts der Innenkanüle (19) verringerten Umfang aufweist.

5. Trachealkanüle nach einem der vorhergehenden Ansprüche, dadurch gekennzeichnet, daß die elastische Membran (29) in ihrem unterhalb der Exspirationsöffnung (16) gelgenen Bereich (32) in entspanntem Zustand etwa einen dem Wölbungsumfang des Querschnitts der Innenkanüle (19) entsprechenden Umfang aufweist.

6. Trachealkanüle nach einem der vorhergehenden Ansprüche, dadurch gekennzeichnet, daß die Innenkanüle (19) ein radiales Anschlagelement (22) zur Begrenzung der Einschubbewegung in die Kanüle (1) aufweist.

7. Trachealkanüle nach Anspruch 6 mit einem Konnektor (20), der mit der Innenkanüle (19) verbunden ist und als Anschlußstück für ein Beatmungsgerät dient, dadurch gekennzeichnet, daß das radiale Anschlagelement (22) an dem Konnektor (20) angeformt ist.

8. Trachealkanüle nach einem der vorhergehenden Ansprüche, dadurch gekennzeichnet, daß auf dem distalen, oberhalb der Krümmung (3) gelegenen Bereich (5) der Kanüle (1) ein ringförmiger Haltesteg (7) verschieb- sowie fixierbar angeordnet ist.

## Claims

1. Tracheal tube for the artificial respiration of tracheotomised patients, having a cuff (10) surrounding the proximal part (4) insertable concentrically in the trachea so as to seal the trachea and a bent region (3) disposed above same, as well as an inner tube (19) insertable concentrically into the tube (1) from its distal end (6), wherein in the tube (1) above the cuff (10) sealing the trachea and roughly in the extension of the longitudinal axis (17) of the bent part (4) insertable into the trachea an expiration aperture is provided, characterised in that the inner tube (19) has in its region below the expiration aperture (16) a recess (26) at least partly covered by an elastic membrane (29) acting as a valve as well as a region of narrowed cross-section at its proximal end (25).

2. Tracheal tube according to claim 1, characterised in that the recess (26) in the inner tube (19) covered with an elastic membrane (29) is continued in a slot-like manner up to its proximal end face (28).

3. Tracheal tube according to claim 2, characterised in that the slot-like recess (26) in the inner tube covered by an elastic membrane assumes in cross-section roughly a semi-circle corresponding to half the cross-section of the inner tube (19).

4. Tracheal tube according to claim 3, characterised in that the elastic membrane (29) has in its region proximal to the expiration aperture (16) in the substantially relaxed state a circumference which is reduced compared to the curved circumference of the cross-section of the inner tube (19).

5. Tracheal tube according to one of the preceding claims, characterised in that the elastic membrane (29) has in its region (32) below the expiration aperture (16) in the relaxed state a circumference corresponding approximately to the curved circumference of the cross-section of the inner tube (19).

6. Tracheal tube according to one of the preceding claims, characterised in that the inner tube (19) has a radial stop element (22) to limit the insertion motion into the tube (1).

7. Tracheal tube according to claim 6, having a connector (20), which is connected to the inner tube (19) and acts as a connection member for a ventilator, characterised in that the radial stop element (22) is integrally formed with the connector (20).

8. Tracheal tube according to one of the preceding claims, characterised in that on the distal region (5) of the tube (1) located above the curvature (3) an annular holding web (7) is displaceably and fixably disposed.

## Revendications

1. Canule trachéale pour la respiration artificielle de patients trachéotomisés, comportant un manchon (10) qui entoure le tronçon proximal (4), insérable concentriquement dans la trachée, et qui ferme cette dernière de façon étanche, et une partie coudée (3), qui est disposée au-dessus de ce manchon, ainsi qu'une canule intérieure (19), qui peut être insérée concentriquement dans la canule (1) à partir de l'extrémité distale (6) de cette dernière, une ouverture d'expiration (16) étant prévue dans la canule (1) au-dessus du manchon (10) qui ferme de façon étanche la trachée, ainsi que sensiblement dans le prolongement de l'axe longitudinal (17) du tronçon recourbé (4) insérable dans la trachée, caractérisée en ce que la canule intérieure (19) comporte, dans sa partie située au-dessous de l'ouverture d'expiration (16), un évidement (26) couvert, au moins partiellement, par une membrane élastique (29) agissant en tant que valve, ainsi qu'une partie de section transversale rétrécie, au niveau de son extrémité proximale (25).

2. Canule trachéale selon la revendication 1, caractérisée en ce que l'évidement (26), couvert par une membrane élastique (29), de la canule intérieure (19) se prolonge en forme de fente jusqu'à la face frontale proximale (28) de cette dernière.

3. Canule trachéale selon la revendication 2, caractérisée en ce que l'évidement en forme de fente (26) de la canule intérieure (19), qui est couvert par une membrane élastique (29), occupe en coupe transversale, à l'extrémité proximale (25), approximativement la moitié du pourtour cintré de la section transversale de la canule intérieure (19).

4. Canule trachéale selon la revendication 3, caractérisée en ce que la membrane élastique (29) présente, dans sa partie placée du côté proximal par rapport à l'ouverture d'expiration (16), dans un état largement détendu, un pourtour réduit par rapport au pourtour incurvé de la section transversale de la canule intérieure (19).

5. Canule trachéale selon l'une des revendications précédentes, caractérisée en ce que la membrane élastique (29) présente à peu près, dans sa partie (32) placée au-dessus de l'ouverture d'expiration (16), dans l'état détendu, un pourtour qui correspond au pourtour incurvé de la section transversale de la canule intérieure (19).

6. Canule trachéale selon l'une des revendications précédentes, caractérisée en ce que la canule intérieure (19) possède un élément de butée radial (22) servant à limiter le mouvement d'enfoncement dans la canule (1).

7. Canule trachéale selon la revendication 6, comportant un connecteur (20), qui est relié à la canule intérieure (19) et sert d'élément de raccordement pour un appareil respiratoire, caractérisée en ce que l'élément de butée radial (22) est formé en une seule pièce sur le connecteur.

8. Canule trachéale selon l'une des revendications précédentes, caractérisée en ce qu'une barrette annulaire de retenue (7) est disposée, en pouvant y être déplacée et immobilisée sur la partie distale (5), située au-dessus du coude (3), de la canule (1).
